# EUROPÄISCHE PATENTSCHRIFT

(11) **EP 2 655 043 B1**
(45) Veröffentlichungstag und Bekanntmachung des Hinweises auf die Patenterteilung: **02.08.2017**
(21) Anmeldenummer: 11805501.1
(22) Anmeldetag: 21.12.2011
(51) Int. Cl.: B29C 65/16, B29C 65/82, G01B 11/06, G01B 17/02

(54) **VERFAHREN ZUR QUALITÄTSSICHERUNG VON GESCHWEISSTEN KUNSTSTOFFBAUTEILEN**
METHOD FOR ENSURING THE QUALITY OF WELDED PLASTICS COMPONENTS
PROCÉDÉ DE CONTRÔLE DE LA QUALITÉ DE COMPOSANTS PLASTIQUES SOUDÉS

(30) Priorität: 21.12.2010 DE 102010055294
(43) Veröffentlichungstag der Anmeldung: 30.10.2013
(73) Patentinhaber: bielomatik Leuze GmbH + Co. KG, 72639 Neuffen (DE)
(72) Erfinder: HEPP, Franz, 72581 Dettingen (DE)
(74) Vertreter: Hoeger, Stellrecht & Partner Patentanwälte mbB
(86) Internationale Anmeldenummer: PCT/EP2011/073658
(87) Internationale Veröffentlichungsnummer: WO 2012/085131

(56) Entgegenhaltungen:
- JP-A- 11 147 258
- JP-A- 2004 001 071

## Beschreibung

Die Erfindung betrifft ein Verfahren zur Feststellung, ob zwei in einer Fügeebene miteinander zu verschweißende Kunststoffbauteile nach Durchführung eines Schweißvorganges miteinander verschweißt sind, gemäß den Merkmalen des Oberbegriffes des Patentanspruches 1.

Grundlage für die Erfindung ist das an sich bekannte Laserdurchstrahlschweißverfahren, bei dem zwei aus einem Kunststoffmaterial bestehende Bauteile im Bereich einer Fügeebene zur Anlage gebracht werden. Anschließend erfolgt ein Schweißvorgang mittels eines Laserstrahles, der die Grenzflächen der Bauteile im Bereich der Fügeebene aufschmilzt und es dadurch nach dem Erkalten zu einer unlösbaren Verbindung der beiden Bauteile miteinander kommt. Hierzu ist es erforderlich, dass das eine Bauteil für den Laser transparent und das andere Bauteil für den Laser, das heißt für den Laserstrahl intransparent oder absorbierend ist.

Je nach Anwendungsfall des fertigen Gesamtbauteiles, das zumindest aus den beiden miteinander zu verschweißenden Kunststoffbauteilen besteht, sind hohe Festigkeits- oder auch Sicherheitsanforderungen an das Endprodukt gestellt. Daher ist es erforderlich, nicht nur den Schweißvorgang zum Zusammenfügen der beiden Kunststoffbauteile auszuführen, sondern während bzw. nach der Durchführung des Schweißvorganges eine Qualitätssicherung durchzuführen.

Für eine solche Qualitätssicherung schlägt die DE10 2005 000 002 A1 ein Verfahren zur Detektion von termischer Schädigung beim Laserdurchstrahlschweißen und eine korrespondierende Vorrichtung zur Durchführung dieses Verfahrens vor. Mit diesem bekannten Verfahren kann beim Laserdurchstrahlschweißen von Kunststoffen die mögliche Entstehung von Verbrennungen auf der Strahleintrittsseite des einen Kunststoffbauteiles prozessbegleitend detektiert werden. Hierzu ist vorgesehen, dass die von der Verbrennung ausgehende Strahlung durch ein entsprechendes Element (Sensor) detektiert und somit ein Ausschleusen eines geschädigten Bauteiles im Rahmen der Serienherstellung solcher Bauteile zu ermöglichen.

Dieses bekannte Verfahren hat jedoch den Nachteil eines aufwendigen und damit komplexen Aufbaus, so dass es für die Serienfertigung und insbesondere für die Qualitätssicherung von in Serienproduktion hergestellten Kunststoffbauteilen nicht oder nur bedingt geeignet ist.

Die JP 11-147258 A offenbart ein Verbindungsverfahren zum Verbinden von zwei Werkstücken, wobei eine Verfahrensüberwachung mittels Ultraschall vorgesehen ist.

Die JP 2004-1071 A offenbart ein Laserdurchstrahlschweißverfahren, wobei das Position eines Absorptionsmaterial mittels Ultraschall ermittelt ist. Der Erfindung liegt die Aufgabe zugrunde, ein gattungsgemäßes Verfahren zur Feststellung, ob zwei in einer Fügeebene miteinander zu verschweißende Kunststoffbauteile nach Durchführung eines Schweißvorganges miteinander verschweißt sind oder nicht, hinsichtlich der Erfassungsgenauigkeit und der Reaktionszeit zu verbessern.

Diese Aufgabe ist durch die Merkmale des Patentanspruches 1 gelöst. Erfindungsgemäß ist vorgesehen, dass vor der Durchführung des Schweißvorganges von einem Referenzpunkt aus die Lage einer Bezugsebene ermittelt, anschließend der Schweißvorgang durchgeführt und während oder nach Beendigung des Schweißvorganges von dem Referenzpunkt aus die Lage der Fügeebene oder die Dicke der miteinander verschweißten Bauteile ermittelt wird. Vor dem Verschweißen von zwei Bauteilen, eines mit der Dicke d1 und eines mit der Dicke d2, existiert immer zwischen diesen beiden Bauteilen eine Grenzfläche, bei der es sich um die einander zugewandte Fügefläche des jeweiliges Bauteiles handelt, wobei in dem Bereich der Fügefläche die beiden Bauteile miteinander mittels des

Laserdurchstrahlschweißverfahrens unlösbar miteinander verbunden werden sollen. Diese Grenzfläche wird im Folgenden als Fügeebene bezeichnet. Vor der Durchführung des Schweißvorganges ist diese Ebene dadurch gekennzeichnet, dass die Oberfläche der beiden einander zugewandten Bauteile nahezu vollständig oder nur unter eines zu vernachlässigenden Spaltes aneinander anliegen. Erfindungsgemäß ist vorgesehen, dass ausgehend von einem Referenzpunkt aus die Lage einer Bezugsebene ermittelt wird. Diese Bezugsebene kann die Fügeebene zwischen den beiden einander zugewandten Fügeflächen der Bauteile sein. Es kann sich hierbei aber auch um die Oberfläche des einen oder des anderen Bauteiles handeln, die auf der der Fügeebene abgewandten Seite liegt. Diese Oberfläche kann auch als freie, weil zugängliche Oberfläche bezeichnet werden. Anschließend wird der Schweißvorgang durchgeführt, wodurch sich im Bereich der Fügeebene die einander zugewandten Oberflächen der beiden Bauteile aufheizen, aufschmelzen und miteinander verschmelzen, so dass daraus keine klar abgegrenzte Fügeebene resultiert, sondern ein Fügebereich entsteht. Dieser Fügebereich ist hinsichtlich seiner Struktur nicht definiert von dem ursprünglichen Material der beiden Bauteile abgrenzbar, so dass ein fließender Übergang von dem ursprünglichen Material vor der Durchführung des Schweißvorganges und dem Fügebereich (nach Durchführung des Schweißvorganges) entsteht. Dieser Fügebereich wird auch als Schweißnahtpenetration bezeichnet. Weiterhin wird erfindungsgemäß während oder nach Beendigung des Schweißvorganges von dem Referenzpunkt aus die Lage der Fügeebene (genauer des Fügebereiches) oder die Dicke der beiden miteinander verschweißten Bauteile ermittelt. Denn nach dem Verschweißen verschmilzt die Fügeebene, woraus resultiert, dass sich die gemessene Dicke Dn des Bauteiles verändert. Diese Veränderung ergibt sich aus dem Wert der beiden Einzeldicken der Bauteile, die als d1 und d2 bezeichnet werden. Vor dem Fügevorgang betrug die icke Dv = d1 + d2. Nach der Durchführung des Schweißvorganges (ggf. auch schon während der Durchführung des Schweißvorganges) kann die Gesamtdicke Dn aus einer der beiden Einzeldicken d1 oder d2 unter Berücksichtigung der Schweissnahtpenetration s gewonnen werden. Der ermittelte Wert für die Lage der Fügeebene bzw. des Fügebereiches nach der Durchführung des Schweißvorganges bzw. die Ermittlung der Dicke der beiden miteinander verschweißten Bauteile ist innerhalb vorgebbarer Toleranzen ein Maß dafür, ob die zwei in der Fügeebene miteinander zu verschweißenden Kunststoffbauteile nach der Durchführung des Schweißvorganges sollgemäß miteinander verschweißt sind oder ob ein Fehler aufgetreten ist. Je nach dem, welches Maß für die Lage der Fügeebene nach Durchführung des Schweißvorganges bzw. welche Dicke der miteinander verschweißten Bauteile ermittelt wurde, kann daraus eine Information abgeleitet werden, ob es sich um einen Fehler (zum Bespiel Lunker) in einem der beiden Kunststoffbauteile (zum Beispiel infolge eines Spritzgießfehlers) oder einen Fehler in der Schweißnaht (folglich eine nicht oder nur teilweise verbundene Grenzfläche) handelt.

Infolgedessen sind mit dem erfindungsgemäßen Verfahren schnelle Messvorgänge und daraus resultierend kurze Messzeiten möglich, die während und/oder nach der Durchführung des Schweißvorganges ausgeführt werden können. Aufgrund der Genauigkeit bei der Bestimmung der Bezugsebene, der Fügeebene sowie der Dicke der miteinander verschweißten Bauteile, jeweils ausgehend von dem Referenzpunkt aus, ist eine sehr genaue Aussage dahingehend möglich, ob der Schweißvorgang zur Verbindung der beiden Kunststoffbauteile miteinander sollgerecht durchgeführt wurde oder ob es sich um einen fehlerhaften Prozess handelt.

Um die schnellen Messzeiten zu ermöglichen, ist in Weiterbildung der Erfindung vorgesehen, dass die Ermittlung der Lage der Fügeebene und der Dicke der miteinander verschweißten Bauteile mittels einer Laserstrahlmessung erfolgt. Um die Dicke der miteinander verschweißten Bauteile zu ermitteln, kann alternativ dazu auch eine Ultraschallmessung erfolgen. Dadurch sind verschiedene Messmöglichkeiten vorgeschlagen, die auch unterschiedliche Einrichtungen (zum Beispiel Laserstrahlquelle, Ultraschallquelle, entsprechende Sensoren und dergleichen) bedingen, wobei diese optimal an die verwendeten Materialien der Kunststoffbauteile und deren Geometrie angepasst werden kann.

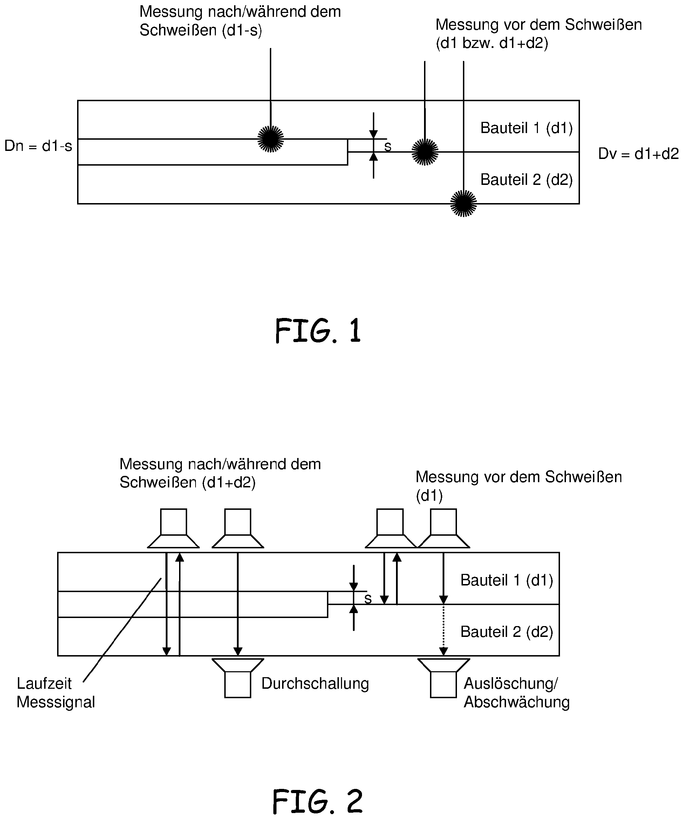

Die Erfindung ist zur weiteren Erläuterung im Folgenden näher beschrieben, wozu auf die beiden Figuren 1 und 2 verwiesen wird.

Figur 1 zeigt als Ausführungsbeispiel eine Laserstrahlmessung, wobei ausgehend von einem Referenzpunkt aus (zum Beispiel die Anordnung der Laserstrahlquelle) die Dicke d1 des Bauteiles 1 und die Dicke d2 des Bauteiles 2 gemessen wird. Das heißt, dass die der Laserstrahlquelle abgewandte Oberfläche des Bauteiles 1 eine erste Bezugsebene und die der Laserstrahlquelle abgewandte Oberfläche des Bauteiles 2 eine weitere Bezugsebene ist, die vor dem Schweißvorgang ermittelt werden. Anschließend wird der Schweißvorgang durchgeführt und die beiden Bauteile 1 und 2 in ihrer Fügeebene miteinander verschweißt. Infolge der Verschweißung verschwindet die klar abgegrenzte Fügeebene, so dass daraus ein Fügebereich wird. Dieser Fügebereich wird ebenfalls ausgehend von dem durch Dn - s. Referenzpunkt aus unter Berücksichtigung der Schweißnahtpenetration s ermittelt. Alternativ oder ergänzend dazu kann auch die entstandene Gesamtdicke Dn des Gesamtbauteiles (bestehend aus den zusammengefügten Bauteilen 1 und 2) ermittelt werden. Diese entstandene Gesamtdicke Dn ist im Regelfall infolge des Fügeprozesses etwas geringer als die ursprüngliche Einzeldicke d1 und d2 der beiden Bauteile 1 und 2. Folglich ist die Differenz zwischen der Addition Dv der Einzeldicken d1 und d2 vor dem Schweißvorgang und die nach dem Schweißvorgang entstandene Gesamtdicke Dn ein Maß für einen ordnungsgemäß durchgeführten oder einen fehlerhaften Schweißvorgang.

Alternativ zu der Laserdistanzmessung ist in Figur 2 eine Ultraschalldistanzmessung vorgesehen. Auch hier gilt wieder das gleiche, wie schon zu der Laserdistanzmessung gemäß Figur 1 ausgeführt. Zunächst werden vor der Durchführung des Schweißvorganges mit entsprechenden Mitteln eine Ultraschalldistanzmessung durchgeführt, um die Einzeldicken d1 und d2 der beiden Bauteile 1 und 2 zu ermitteln. Dies erfolgt entweder dadurch, dass ein Ultraschall in das eine Bauteil eingekoppelt, am zweiten Bauteil reflektiert, zurückgestrahlt und wieder ausgekoppelt wird, wobei die Laufzeit ein Maß für die Dicke d1 des Bauteiles 1 ist. Die Gesamtdicke Dv vor dem Schweißvorgang kann beispielsweise dadurch ermittelt werden, dass in die Oberfläche des ersten Bauteiles 1 ein Ultraschallsignal eingespeist, beide Bauteile 1 und 2 durchläuft und an der Oberfläche des zweiten Bauteiles 2 wieder ausgekoppelt wird. Auch hier ist die Laufzeit für das Ultraschallsignal durch die beiden Bauteile ein Maß für die Dicke der beiden Bauteile 1 und 2 vor dem Schweißvorgang. Gleiches gilt für die Dickenmessung nach der Durchführung des Schweißvorganges. Auch hier kann eine Laufzeitmessung oder alternativ eine Durchschallung erfolgen. Dies ist in Figur 2 in der linken Hälfte durch den Schnitt der beiden Bauteile 1 und 2 dargestellt. Auch hier kann aus der ermittelten Gesamtdicke D vor dem Schweißvorgang (Figur 2, rechte Hälfte) und der sich nach der Durchführung des Schweißvorganges ergebenden neuen Gesamtdicke D (bei Betrachtung der Figur 2 auf der linken Seite) ein Maß erhalten werden, aus dem abgeleitet werden kann, ob der Schweißvorgang, insbesondere das Laserdurchstrahlschweißen, erfolgreich ausgeführt wurde (so dass die beiden Kunststoffbauteile sollgerecht miteinander verbunden worden sind) oder ob ein Fehler aufgetreten ist (so dass dieses entstandene Gesamtbauteil nicht den Anforderungen entspricht und beispielsweise bei einer Serienproduktion aus dieser ausgeschleust werden muss).

Im folgenden wird die Erfindung noch einmal mit anderen Worten wiedergegeben.

Vor dem Verschweißen von zwei Bauteilhälften der Dicke 1 (d1) und der Dicke 2 (d2) existiert immer zwischen den zwei Bauteilen eine Grenzfläche (Fügeebene).

Diese Grenzfläche kann erfindungsgemäß entweder durch
- Laserabstandsmessung (bei lasertransparenten Bauteilen, bzw. bei der Verschweißung eines strahlungstransparenten mit einem strahlungsabsorbierenden Bauteil) oder durch
- Ultraschall (im Impulsechoverfahren durch Messung der Schalllaufzeiten im Kunststoff) lokalisiert werden.

Nach dem Verschweißen verschmilzt die Grenzfläche und die gemessene Dicke des Bauteils verändert sich:
- bei Laserabstandsmessung zweier transparenter Bauteile auf den Wert d1 + d2
- bei Laserabstandsmessung eines transparenten und eines absorbierenden Bauteils auf den Wert d1-s (wobei s = Schweißnahtpenetration in das transparente Teil → die Schmelzen vermischen sich und ein Teil des absorbierenden Materials wird auch absorbierend, so reduziert sich die gemessene Dicke)
- bei Ultraschalldickenmessung auf d1+d2

Die Vorteile des Verfahrens sind:
- schnelles Messen, kurze Messzeiten
- Information über die Tiefe des Fehlers macht Interpretation möglich, ob es sich um einen Lunker im Bauteil (Spritzgießfehler) oder einen Fehler in der Schweißnaht (nicht verbundene Grenzfläche) handelt

Verfahrensvarianten:
Laserdistanzmessung mit Lasern bzw. E, deren Wellenlänge so ist, dass der Kunststoff in diesem Wellenlängenbereich transparent ist,

Anpassung der Laserart/-Wellenlänge und der Ergebnisauswertung an die optischen Eigenschaften des verwendeten Kunststoffes (Absorption, optische Eindringtiefe),
Ultraschallmessung mit aufgesetztem Ultraschallsensor,
Ultraschallmessung durch die Luft mit nicht berührendem Ultraschallsensor,
Ultraschallmessung im Durchschallungsverfahren

## Patentansprüche

1. Verfahren zur Feststellung, ob zwei in einem Laserdurchstrahlschweißverfahren in einer Fügeebene miteinander zu verschweißende Kunststoffbauteile nach Durchführung eines Schweißvorganges miteinander verschweißt sind, wobei eines der Kunststoffbauteile für einen Laserstrahl transparent ist, wobei ein weiteres der Kunststoffbauteile für den Laserstrahl intransparent oder absorbierend ist, und wobei vor der Durchführung des Schweißvorganges von einem Referenzpunkt aus die Lage einer Bezugsebene ermittelt, anschließend der Schweißvorgang durchgeführt und während oder nach Beendigung des Schweißvorganges von dem Referenzpunkt aus die Lage der Fügeebene mittels einer Laserstrahlmessung ermittelt wird.

2. Verfahren nach Anspruch 1, **dadurch gekennzeichnet, dass** die Ermittlung der Dicke der miteinander verschweißten Bauteile mittels einer Laserstrahlmessung erfolgt.

3. Verfahren nach Anspruch 1, **dadurch gekennzeichnet, dass** die Ermittlung der Dicke der miteinander verschweißten Bauteile mittels einer Ultraschallmessung erfolgt.

4. Verfahren nach einem der vorhergehenden Ansprüche, **dadurch gekennzeichnet, dass** die Lage der Bezugsebene vor dem Schweißvorgang ein Maß für die Dicke d1 des einen Kunststoffbauteiles ist.

5. Verfahren nach einem der vorhergehenden Ansprüche, **dadurch gekennzeichnet, dass** die Lage der Bezugsebene nach dem Schweißvorgang unter Berücksichtigung eines Maßes (s) für die Schweißnahtpenetration ermittelt wird.

6. Verfahren nach Anspruch 5, **dadurch gekennzeichnet, dass** die Ermittlung der Bezugsebene nach dem Schweißvorgang mittels Laserstrahlmessung durch das für den Laserstrahl transparente Kunststoffbauteil erfolgt.

7. Verfahren nach einem der vorhergehenden Ansprüche, **dadurch gekennzeichnet, dass** die Gesamtdicke d1 + d2 der miteinander verschweißten Kunststoffbauteiles mittels Ultraschall-Laufzeitmessung erfolgt, wobei der Ultraschall nach dem Durchlauf durch die miteinander verschweißten Kunststoffbauteile reflektiert wird und die miteinander verschweißten Kunststoffbauteile erneut durchläuft.

8. Verfahren nach einem der vorhergehenden Ansprüche, **dadurch gekennzeichnet, dass** die Gesamtdicke d1 + d2 der miteinander verschweißten Kunststoffbauteiles mittels Ultraschall-Durchschallung erfolgt, wozu die miteinander verschweißten Kunststoffbauteile für Ultraschall durchlässig sind.

9. Verfahren nach einem der vorhergehenden Ansprüche, **dadurch gekennzeichnet, dass** die Laserart und/oder die Wellenlänge des Lasers an den Kunststoff der Bauteile angepasst ist.

## Claims

1. A method for determining whether two plastics components to be welded to each other in a joint plane by means of laser transmission welding are welded to each other after a welding process has been carried out, wherein one of the plastics components is transparent to a laser beam, wherein a further plastics component is nontransparent or absorbent to the laser beam, and wherein before the welding process is carried out, the location of a reference plane is ascertained from a reference point, subsequently the welding process is carried out, and during or after the ending of the welding process, the location of the joint plane is ascertained from the reference point by means of a laser beam measurement.

2. The method as claimed in claim 1, **characterized in that** the ascertainment of the thickness of the components welded to one another is performed by means of a laser beam measurement.

3. The method as claimed in claim 1, **characterized in that** the ascertainment of the thickness of the components welded to one another is performed by means of an ultrasonic measurement.

4. The method as claimed in any one of the preceding claims, **characterized in that** the location of the reference plane before the welding process is a measure of the thickness d1 of one plastics component.

5. The method as claimed in any one of the preceding claims, **characterized in that** the location of the reference plane after the welding process is ascertained in consideration of a measure (s) for the weld seam penetration.

6. The method as claimed in claim 5, **characterized in that** the ascertainment of the reference plane after the welding process is performed by means of laser beam measurement through the plastics component transparent to the laser beam.

7. The method as claimed in any one of the preceding claims, **characterized in that** the total thickness d1 + d2 of the plastics components welded to one another is performed by means of ultrasonic runtime measurement, wherein the ultrasound is reflected after passing through the plastics components welded to one another, and again passes through the plastics components welded to one another.

8. The method as claimed in any one of the preceding claims, **characterized in that** the total thickness d1 + d2 of the plastics components welded to one another is performed by means of ultrasonic through transmission, for which purpose the plastics components welded to one another are transmissive to ultrasound.

9. The method as claimed in any one of the preceding claims, **characterized in that** the type of laser and/or the wavelength of the laser is adapted to the plastic of the components.

## Revendications

1. Procédé pour déterminer si deux composants plastiques à souder l'un à l'autre dans un plan de joint dans un procédé de soudage par faisceau laser sont soudés l'un à l'autre après réalisation d'un processus de soudage, dans lequel un des composants plastiques est transparent à un faisceau laser, dans lequel un autre des composants plastiques est non transparent ou absorbant au faisceau laser, et dans lequel la position d'un plan de référence est déterminée avant la réalisation du processus de soudage à partir d'un point de référence, puis le processus de soudage est réalisé et la position du plan de joint est déterminée pendant ou après la fin du processus de soudage à partir du point de référence au moyen d'une mesure par faisceau laser.

2. Procédé selon la revendication 1, **caractérisé en ce que** la détermination de l'épaisseur des composants soudés l'un à l'autre se fait au moyen d'une mesure par faisceau laser.

3. Procédé selon la revendication 1, **caractérisé en ce que** la détermination de l'épaisseur des composants soudés l'un à l'autre se fait au moyen d'une mesure par ultrasons.

4. Procédé selon l'une quelconque des revendications précédentes, **caractérisé en ce que** la position du plan de référence avant le processus de soudage est une dimension pour l'épaisseur d1 de l'un composant plastique.

5. Procédé selon l'une quelconque des revendications précédentes, **caractérisé en ce que** la position du plan de référence après le processus de soudage est déterminé en tenant compte d'une dimension (s) pour la pénétration du cordon de soudure.

6. Procédé selon la revendication 5, **caractérisé en ce que** la détermination du plan de référence se fait après le processus de soudage au moyen de la mesure par faisceau laser par le composant plastique transparent au faisceau laser.

7. Procédé selon l'une quelconque des revendications précédentes, **caractérisé en ce que** l'épaisseur totale d1 + d2 des composants plastiques soudés l'un à l'autre se fait au moyen d'une mesure du temps de propagation d'ultrasons, dans lequel les ultrasons sont réfléchis après le passage par les composants plastiques soudés l'un à l'autre et traversent à nouveau les composants plastiques soudés l'un à l'autre.

8. Procédé selon l'une quelconque des revendications précédentes, **caractérisé en ce que** l'épaisseur totale d1 + d2 des composants plastiques soudés l'un à l'autre se fait au moyen d'un sondage par ultrasons, ce pour quoi les composants plastiques soudés l'un à l'autre sont perméables aux ultrasons.

9. Procédé selon l'une quelconque des revendications précédentes, **caractérisé en ce que** le type de laser et/ou la longueur d'ondes du laser est adapté au plastique des composants.
